# EUROPÄISCHE PATENTSCHRIFT

(11) **EP 0 613 858 B1**
(45) Veröffentlichungstag und Bekanntmachung des Hinweises auf die Patenterteilung: **14.05.1997**
(21) Anmeldenummer: 94101680.0
(22) Anmeldetag: 04.02.1994
(51) Int. Cl.: C01B 25/32, A61K 7/16

(54) **Verfahren zur Herstellung eines für den Einsatz in Zahnpastengeeigneten Dicalciumphosphat-Dihydrats**
Process for the preparation of dicalcium phosphate dihydrate suitable for an application in the toothpastes
Procédé de préparation de phosphate dicalcique dihydraté convenant à une application dans les dentifrices

(30) Priorität: 04.03.1993 DE 4306673
(43) Veröffentlichungstag der Anmeldung: 07.09.1994
(73) Patentinhaber: HOECHST AKTIENGESELLSCHAFT, 65926 Frankfurt am Main (DE)
(72) Erfinder: Dany, Franz-Josef, Dr., D-50374 Erftstadt (DE); Kalteyer, Gerhard, D-50374 Erfstadt (DE); Nolte, Gerhard, Dr., D-50374 Erftstadt (DE); Prell, Hedwig, D-50354 Hürth (DE)

(56) Entgegenhaltungen:
- EP-A- 0 045 826
- EP-A- 0 240 880
- DE-A- 2 648 061

## Beschreibung

Die Erfindung betrifft ein Verfahren zur Herstellung von Dicalciumphosphat-Dihydrat mit guter Fluorverträglichkeit und Hydrolysestabilität, das bei seiner Anwendung in Zahnpasten eine möglichst geringe Nachdickung bewirkt, durch Umsetzung von in Wasser suspendiertem Calciumcarbonat mit einer wäßrigen Lösung von Orthophosphorsäure in einer ersten Fällstufe, Auffällung von Dimagnesiumphosphat-Trihydrat als Stabilisator durch Umsetzung wäßriger Lösungen eines Magnesiumsalzes und von Orthophosphorsäure in Gegenwart einer basischen Verbindung in einer zweiten Fällstufe, sowie abschließendes Abfiltrieren, Trocknen und Mahlen des Niederschlags.

Ein derartiges "Kurzzeitverfahren" ist im wesentlichen in der DE 26 48 o61 C2 (= GB 1 548 465) beschrieben. Dicalciumphosphat-Dihydrat (DCP-D) und Dimagnesiumphosphat-Trihydrat (DMP-T) werden auch als Calciumhydrogenphosphat-Dihydrat bzw. Magnesiumhydrogenphosphat-Trihydrat bezeichnet. Die Bedeutung der Fluorverträglichkeit und Hydrolysestabilität ist in der DE 26 48 o61 C2, Spalte 2, erläutert. Das Verfahren der DE 26 48 o61 C2 befriedigt nicht bezüglich des Viscositätsverhaltens der aus dem stabilisierten DCP-D hergestellten Zahnpasten. Die Nachdickung der Zahnpasten, die mit einem derart hergestellten DCP-D zustande kommt und in hohem Maße unerwünscht ist, wird u.a. durch einen Verlust des DCP-D an Kristallwasser und durch seinen hydrolytischen Abbau zu Calciumhydroxylapatit und Orthophosphorsäure verursacht. Diese unerwünschten Reaktionen bewirken gleichzeitig in fluorhaltigen Zahnpasten über die intermediäre Bildung von Calciumfluorid und schließlich von Fluorapatit einen hohen Verlust an kariesprophylaktischen, aktiven Fluorionen.

Weiterhin ist aus der DE 32 46 884 C2 (= US-A-4,496,527) ein Verfahren zur Herstellung von DCP-D bekannt, wobei man eine Löschkalktrübe mit Phosphorsäure zu einer Lösung von DCP-D umsetzt, die man mit einer solchen Menge an zusätzlicher Löschkalktrübe und mit Pyrophosphorsäure versetzt, daß man eine DCP-D-Trübe mit einem pH-Wert zwischen 2,2 und 4,9 erhält, und das erhaltene DCP-D aus der Trübe abtrennt. Die Pyrophosphorsäure wird in einer Menge von o,1 bis 1,o Gew%, berechnet auf das herzustellende DCP-D, zugesetzt. Als Stabilisator kann auch fertiges DMP-T zugesetzt werden, beispielsweise zur Reaktionsmaische vor dem Abtrennen des DCP-D.

Das Verfahren der DE 32 46 884 C2 hat jedoch den Nachteil, daß die vollständige Umsetzung der Löschkalktrübe mit den verwendeten Phosphorsäuren mindestens die 2ofache Zeit benötigt wie bei dem in der DE 26 48 o61 C2 beschriebenen Kurzzeitverfahren. Der Nachweis der unvollständigen Umsetzung der Löschkalktrübe bei kürzeren Reaktionszeiten läßt sich dadurch erbringen, daß man eine Probe der Fällungstrübe mit einer Phenolphthaleinlösung versetzt, welche die nicht umgesetzten Calciumhydroxydpartikel als rote Punkte in der Trübe indiziert.

Die Nachteile des geschilderten Standes der Technik lassen sich weitgehend vermeiden, wenn man beim eingangs beschriebenen Kurzzeitverfahren gemäß vorliegender Erfindung dem Reaktionsgemisch als weiteren Stabilisator o,2 bis o,8 Gew% Polyphosphorsäure, berechnet auf das im Reaktionsgemisch niedergeschlagene Dicalciumphosphat-Dihydrat, zusetzt, wobei die Polyphosphorsäure in der zweiten Fällstufe gegen Ende der Auffällung von Dimagnesium-Trihydrat eingesetzt wird.

Darüberhinaus ist das Verfahren der Erfindung bevorzugt und wahlweise dadurch gekennzeichnet, daß
a) man Polyphosphorsäure mit einem Phosphorgehalt entsprechend 78 Gew% Phosphorpentoxid einsetzt;
b) man annähernd stöchiometrische Mengen der Calciumcarbonat-Suspension und der Orthophosphorsäure-Lösung in ein Reaktionsgefäß eingibt und die Reaktion bei 5o °C nicht übersteigenden Temperaturen in einem pH-Bereich von 2,2 bis 2,6 hält; daß man den pH-Bereich des Reaktionsgemischs mit Natronlauge auf 5,6 bis 5,8 anhebt und unter gleichzeitigem Eintragen stöchiometrischer Mengen wäßriger Lösungen von Magnesiumsalz, Orthophosphorsäure und Natronlauge Dimagnesiumphosphat-Trihydrat in einer Menge von 2 bis 4 Gew%, berechnet auf das im Reaktionsgemisch niedergeschlagene Dicalciumphosphat-Dihydrat, bei einem pH-Wert von 5,6 bis 6,o auffällt; daß man gegen Ende dieser Auffällung dem Reaktionsgemisch 0,2 bis 0,8 Gew% Polyphosphorsäure zusetzt, berechnet auf das im Reaktionsgemisch niedergeschlagene Dicalciumphosphat-Dihydrat, das Reaktionsgemisch 2 bis 3 min nachrührt und gleichzeitig den pH-Wert mit Natronlauge auf den Neutralpunkt bringt; und daß man das ausgefällte Produkt aus dem Reaktionsgemisch abtrennt, trocknet und auf die für die Anwendung in Zahnpasten übliche Korngröße zerkleinert;
c) man die in der zweiten Fällstufe einzutragende stöchiometrische Menge Orthophosphorsäure entsprechend der äquivalenten Acidität der zuzusetzenden Menge Polyphosphorsäure vermindert;
d) das Reaktionsgefäß zwecks Kühlung mit einem Doppelmantel versehen ist.

Als Magnesiumsalz kann z.B. Magnesiumchlorid oder -nitrat eingesetzt werden.

Die nachfolgenden Beispiele sollen das erfindungsgemäße Verfahren zur Herstellung und Stabilisierung von DCP-D verdeutlichen.

### Beispiel 1 (zum Vergleich)

In einem rührbaren, zwecks Kühlung mit Doppelmantel versehenen Edelstahlreaktor (um die Reaktionstemperatur auf maximal 5o °C zu begrenzen) wurden 2 kg Wasser vorgelegt. Dann wurden unter Rühren mittels eines Propellers (13oo UpM) gleichzeitig 1 kg Calciumcarbonat als 4o gew%ige wäßrige Suspension und ein Gemisch von o,9791 kg Orthophosphorsäure als 75 gew%ige wäßrige Lösung und 6,88 g Polyphosphorsäure (78 Gew% Phosphorpentoxid) in der Weise eingetragen, daß der pH-Wert der Reaktionsmischung im Bereich von 2,2 bis 2,6 gehalten wurde. Dann wurde durch Zugabe von 25 gew%iger Natronlauge der pH-Wert auf 5,7 angehoben. Unter gleichzeitigem Eintragen von o,o235 kg Magnesiumchlorid als 33,3 gew%ige wäßrige Lösung, o,o242 kg Orthophosphorsäure als 75 gew%ige wäßrige Lösung und 25 gew%iger Natronlauge wurde der pH-Wert so gesteuert, daß er einen Wert von 6 nicht überschritt. Die Reaktionsmischung wurde 2 bis 3 min nachgerührt und durch weitere Zugabe von Natronlauge wurde der pH-Wert auf den Neutralpunkt angehoben. Der entstandene Niederschlag wurde über eine Porzellannutsche abfiltriert, mit reichlich destilliertem Wasser gewaschen, getrocknet und auf die für die Anwendung in Zahnpasten erforderliche Korngröße zerkleinert.

Die aufgefällte Menge an DMP-T betrug o,o43 kg, entsprechend 2,5 Gew%, die eingetragene Menge Polyphosphorsäure (78 Gew% P₂O₅) o,4 Gew%, jeweils berechnet auf 1,7194 kg ausgefälltes DCP-D.

### Beispiel 2 (zum Vergleich)

Wie Beispiel 1 mit dem Unterschied, daß die Zugabe der 6,88 g Polyphosphorsäure (78 Gew% P₂O₅) nicht zur Orthophosphorsäure, sondern getrennt bei einem pH-Wert von 2,2 bis 2,6 erfolgte, nachdem die Umsetzung zwischen dem Calciumcarbonat und der Orthophosphorsäure praktisch abgeschlossen war. Nach anschließender Anhebung des pH-Wertes auf 5,7 wurde weiter verfahren, wie in Beispiel 1 beschrieben.

### Beispiel 3 (zum Vergleich)

Wie Beispiel 1 mit dem Unterschied, daß die 6,88 g Polyphosphorsäure mit der für die zweite Fällstufe benötigten o,o242 kg Orthophosphorsäure vermischt und mit o,o235 kg Magnesiumchlorid als 33,3 gew%ige wäßrige Lösung unter gleichzeitiger Zugabe von 25 gew%iger Natronlauge zur Umsetzung gebracht wurden. Hierbei wurde durch entsprechende Dosierung der Natronlauge die Reaktion so gesteuert, daß der pH-Wert von 6 nicht überschritten wurde. Nach 2 bis 3 minütigem Nachrühren wurde der pH-Wert mittels Natronlauge auf den Neutralpunkt angehoben.

### Beispiel 4

Wie Beispiel 1 mit dem Unterschied, daß die 6,88 g Polyphosphorsäure am Ende des zweiten Fällungsschrittes getrennt zugegeben wurden. Hierbei wurde der Anteil der Orthophosphorsäure auf o,o2343 kg vermindert und mit o,o235 kg Magnesiumchlorid unter gleichzeitiger Zugabe von Natronlauge dem Reaktionsgemisch in der Weise zugesetzt, daß der pH-Wert von 6 nicht überschritten wurde. Dann erfolgte die getrennte Zugabe von 6,88 g Polyphosphorsäure unter gleichzeitiger Dosierung von Natronlauge bei einem pH-Wert unter 6. Nach 2 bis 3 minütigem Nachrühren wurde der pH-Wert mittels Natronlauge auf den Neutralpunkt gebracht.

### Beispiel 5

Wie Beispiel 4 mit dem Unterschied, daß die für den zweiten Fällungsschritt benötigte Orthophosphorsäure auf o,o2382 kg vermindert wurde und der Anteil der getrennt zugegebenen Polyphosphorsäure 3,44 g betrug.

Die eingebrachte Menge an Polyphosphorsäure (3,44 g) betrug o,2 Gew%, berechnet auf ausgefälltes DCP-D.

### Beispiel 6

Wie Beispiel 4 mit dem Unterschied, daß die Menge an Orthophosphorsäure o,o23o4 kg und die Menge an Polyphosphorsäure 1o,3 g betrug.

Die eingebrachte Menge an Polyphosphorsäure (1o,3 g) betrug o,6 Gew%, berechnet auf ausgefälltes DCP-D.

### Beispiel 7

Wie Beispiel 4 mit dem Unterschied, daß die Menge an Orthophosphorsäure o,o2266 kg und die Menge an Polyphosphorsäure 13,8 g betrug.

Die eingebrachte Menge von Polyphosphorsäure (13,8 g) betrug o,8 Gew%, berechnet auf ausgefälltes DCP-D.

Die gemäß den Beispielen 1 bis 7 gefertigten Produkte wurden hinsichtlich ihres Stabilitätsverhaltens untersucht.

Die Fluorverträglichkeit der einzelnen Produkte wurde nach folgender Methode geprüft:
1o g des zu prüfenden DCP-D werden in 9o g Wasser aufgeschlämmt und auf 8o °C erwärmt. Dann werden 76 mg Natriummonofluorphosphat (Na₂FPO₃, entsprechend 1ooo ppm Fluor, auf eingesetztes DCP-D bezogen) eingebracht. Die Suspension wird unter ständigem Rühren genau 1 Std. bei 8o °C gehalten. Es wird im Eisbad auf Zimmertemperatur abgekühlt, über eine Fritte filtriert und in einem aliquoten Teil des Filtrates der Fluorgehalt bestimmt.

Der Gehalt an löslichem Fluorion ist ein Maß für die Fluorverträglichkeit des DCP-D. Angegeben wird dieser in Gew% des Ausgangswertes.

Zur Bestimmung der Hydrolysestabilität kam folgende Methode zur Anwendung:
In einer Lösung von 1,63 g Natriumfluorid in 1oo ml Wasser, die auf 6o °C erhitzt und auf dieser Temperatur gehalten wird, werden 25 g des zu prüfenden DCP-D suspendiert und mittels eines Rührers in der Schwebe gehalten. Hierbei wird laufend der pH-Wert registriert. Es wird der Zeitpunkt ermittelt, bei dem der pH-Wert unter 4 absinkt. Die Zeit bis zum Erreichen von pH = 4 ist ein Maß für die Hydrolysestabilität des DCP-D.

Das Verhalten der Pastenviskosität wurde in der folgenden Weise untersucht:
Aus den einzelnen DCP-D-Produkten gemäß den Beispielen 1 bis 7 wurden auf Basis folgender Formulierung Zahnpasten gefertigt:
48,o Gew.-Teile DCP-D
24,o Gew.-Teile Sorbit (7o gew%ige wäßrige Lösung)
6,o Gew.-Teile Glycerin
1,5 Gew.-Teile Natriumlaurylsulfat
o,8 Gew.-Teile Binder
o,8 Gew.-Teile Aroma
o,76 Gew.-Teile Natriummonofluorphosphat
o,25 Gew.-Teile Tetranatriumdiphosphat
o,2 Gew.-Teile Natriumsaccharinat
Ad 1oo Gew.-Teile Deionisiertes Wasser

Die auf Basis vorstehender Rezeptur hergestellten Pasten wurden in Aluminiumtuben abgefüllt und 24 Stunden bei 25 °C gelagert. Bei Umgebungstemperatur wurde mittels eines Rotationsviskosimeters (Brookfield RVT DV II-Spindle D) die Viskosität in Skalenteilen (SkT) gemessen. Dann folgte eine Lagerung der Pasten bei 49 °C, wobei nach 3 und nach 9 Wochen die Viskositätsmessung nach Abkühlung auf Umgebungstemperatur wiederholt wurde.

Nachstehend sind zur besseren Übersicht unter Angabe der Herstellungsart die Stabilitäts- und Viskositätsergebnisse der Pasten, die aus den einzelnen DCP-D-Produkten der Beispiele 1 bis 7 gefertigt wurden, tabellarisch zusammengefaßt:

| Beispiel Nr. | 1 | 2 | 3 | 4 | 5 | 6 | 7 |
|---|---|---|---|---|---|---|---|
| MgHPO₄ . 3 H₂O (Gew%) | 2,5 | 2,5 | 2,5 | 2,5 | 2,5 | 2,5 | 2,5 |
| Polyphosphorsäure (Gew%) | 0,4 | 0,4 | 0,4 | 0,4 | 0,2 | 0,6 | 0,8 |
| Art der Stabilisierung *) | A | B | C | D | D | D | D |
| Fluor-Verträglichkeit (Gew% wasserlösl. Fluor) | 76 | 77 | 80 | 82 | 80 | 83 | 83 |
| Hydrolysestabilität (Zeit (h) bis Erreichen von pH = 4) | 9,6 | 10,2 | 12,3 | >20 | 19,3 | >20 | >20 |

| Pastenviskosität (Skalenteile) | | | | | | | |
|---|---|---|---|---|---|---|---|
| nach 24 h bei 25 °C | 23 | 24 | 24 | 24 | 22 | 25 | 26 |
| nach 3 Wochen bei 49 °C | 70 | 69 | 70 | 34 | 36 | 36 | 37 |
| nach 9 Wochen bei 49 °C | 87 | 78 | 74 | 45 | 48 | 47 | 49 |

| | | | | | | | |
|---|---|---|---|---|---|---|---|
| *) A: Zugabe der Polyphosphorsäure im Gemisch mit der Orthophosphorsäure, die für die erste Fällstufe benötigt wird. B: Getrennte Zugabe der Polyphosphorsäure, nachdem die Umsetzung von Calciumcarbonat mit Orthophosphorsäure praktisch abgeschlossen ist, bei einem pH-Wert von 2,2 bis 2,6. C: Zugabe der Polyphosphorsäure im Gemisch mit der Orthophosphorsäure, die für die 2. Fällungsstufe benötigt wird. D: Getrennte Zugabe der Polyphosphorsäure am Ende der 2. Fällungsstufe, nachdem die Orthophosphorsäure-Menge um die äquivalente Acidität der Polyphosphorsäure vermindert wurde. | | | | | | | |

Wie ersichtlich liefern die erfindungsgemäßen Produkte, die gemäß den Beispielen 4 bis 7 hergestellt sind (Stabilisierung D), die mit Abstand besten Ergebnisse in Bezug auf das Viskositätsverhalten der daraus hergestellten Zahnpasten, wobei gleichzeitig auch gute Werte für die Fluorverträglichkeit und die Hydrolysestabilität festgestellt werden.

## Patentansprüche

1. Verfahren zur Herstellung von Dicalciumphosphat-Dihydrat mit guter Fluorverträglichkeit und Hydrolysestabilität, das bei seiner Anwendung in Zahnpasten eine möglichst geringe Nachdickung bewirkt, durch Umsetzung von in Wasser suspendiertem Calciumcarbonat mit einer wäßrigen Lösung von Orthophosphorsäure in einer ersten Fällstufe, Auffällung von Dimagnesiumphosphat-Trihydrat als Stabilisator durch Umsetzung wäßriger Lösungen eines Magnesiumsalzes und von Orthophosphorsäure in Gegenwart einer basischen Verbindung in einer zweiten Fällstufe sowie abschließendes Abfiltrieren, Trocknen und Mahlen des Niederschlags, dadurch gekennzeichnet, daß man dem Reaktionsgemisch als weiteren Stabilisator 0,2 bis 0,8 Gew% Polyphosphorsäure, berechnet auf das im Reaktionsgemisch niedergeschlagene Dicalciumphosphat-Dihydrat, zusetzt, wobei die Polyphosphorsäure in der zweiten Fällstufe gegen Ende der Auffällung von Dimagnesiumphosphat-Trihydrat eingesetzt wird.

2. Verfahren nach Anspruch 1, dadurch gekennzeichnet, daß man Polyphosphorsäure mit einem Phosphorgehalt entsprechend 78 Gew% Phosphorpentoxid einsetzt.

3. Verfahren nach Anspruch 1 oder 2, dadurch gekennzeichnet, daß man annähernd stöchiometrische Mengen der Calciumcarbonat-Suspension und der Orthophosphorsäure-Lösung in ein Reaktionsgefäß eingibt und die Reaktion bei 50 °C nicht übersteigenden Temperaturen in einem pH-Bereich von 2,2 bis 2,6 hält; daß man den pH-Bereich des Reaktionsgemisches mit Natronlauge auf 5,6 bis 5,8 anhebt und unter gleichzeitigem Eintragen stöchiometrischer Mengen wäßriger Lösungen von Magnesiumsalz, Orthophosphorsäure und Natronlauge Dimagnesiumphosphat-Trihydrat in einer Menge von 2 bis 4 Gew%, berechnet auf das im Reaktionsgemisch niedergeschlagene Dicalciumphosphat-Dihydrat, bei einem pH-Wert von 5,6 bis 6,0 auffällt; daß man gegen Ende dieser Auffällung dem Reaktionsgemisch 0,2 bis 0,8 Gew% Polyphosphorsäure zusetzt, berechnet auf das im Reaktionsgemisch niedergeschlagene Dicalciumphosphat-Dihydrat, das Reaktionsgemisch 2 bis 3 min nachrührt und gleichzeitig den pH-Wert mit Natronlauge auf den Neutralpunkt bringt; und daß man das ausgefällte Produkt aus dem Reaktionsgemisch abtrennt, trocknet und auf die für die Anwendung in Zahnpasten üblich Korngröße zerkleinert.

4. Verfahren nach Anspruch 3, dadurch gekennzeichnet, daß man die in der zweiten Fällstufe einzutragende stöchiometrische Menge Orthophosphorsäure entsprechend der äquivalenten Acidität der zuzusetzenden Menge Polyphsophorsäure vermindert.

5. Verfahren nach mindestens einem der vorhergehenden Ansrpüche, dadurch gekennzeichnet, daß das Reaktionsgefäß zwecks Kühlung mit einem Doppelmantel versehen ist.

## Claims

1. A process for the preparation of dicalcium phosphate dihydrate, which has a good compatibility with fluorine and stability to hydrolysis and causes the minimum possible after-thickening when used in toothpastes, by reaction of calcium carbonate suspended in water with an aqueous solution of orthophosphoric acid in a first precipitating stage, precipitation of dimagnesium phosphate trihydrate, as a stabilizer, by reaction of aqueous solutions of a magnesium salt and of orthophosphoric acid in the presence of a basic compound in a second precipitating stage, and final filtering, drying and grinding of the precipitate, which comprises adding to the reaction mixture 0.2 to 0.8 % by weight of polyphosphoric acid, calculated with respect to the dicalcium phosphate dihydrate precipitated in the reaction mixture, as a further stabilizer, the polyphosphoric acid being employed in the second precipitating stage towards the end of the precipitation of dimagnesium phosphate trihydrate.

2. The process as claimed in claim 1, wherein polyphosphoric acid with a phosphorus content corresponding to 78 % by weight of phosphorus pentoxide is employed.

3. The process as claimed in claim 1 or 2, wherein approximately stoichiometric amounts of the calcium carbonate suspension and the orthophosphoric acid solution are introduced into a reaction vessel and the reaction is kept at temperatures which do not exceed 50°C in a pH range of 2.2 to 2.6; wherein the pH range of the reaction mixture is increased to 5.6 to 5.8 with sodium hydroxide solution and dimagnesium phosphate trihydrate is precipitated in an amount of 2 to 4 % by weight, calculated with respect to the dicalcium phosphate dihydrate precipitated in the reaction mixture, at a pH of 5.6 to 6.0 while simultaneously introducing stoichiometric amounts of aqueous solutions of magnesium salt, orthophosphoric acid and sodium hydroxide solution; wherein 0.2 to 0.8 % by weight of polyphosphoric acid, calculated with respect to the dicalcium phosphate dihydrate precipitated in the reaction mixture, is added to the reaction mixture towards the end of this precipitation, the reaction mixture is subsequently stirred for 2 to 3 minutes and the pH is simultaneously brought to the neutral point with sodium hydroxide solution; and wherein the precipitated product is separated off from the reaction mixture, dried and comminuted to the particle size customary for use in toothpastes.

4. The process as claimed in claim 3, wherein the stoichiometric amount of orthophosphoric acid to be introduced in the second precipitating stage is reduced in accordance with the equivalent acidity of the amount of polyphosphoric acid to be added.

5. The process as claimed in at least one of the preceding claims, wherein the reaction vessel is provided with a double jacket for the purpose of cooling.

## Revendications

1. Procédé de production de phosphate dicalcique dihydraté ayant une bonne compatibilité avec le fluor et une bonne stabilité à l'hydrolyse, qui, lors de son utilisation dans des pâtes dentifrices, provoque un post-épaississement aussi faible que possible, par la réaction de carbonate de calcium, mis en suspension dans de l'eau, avec une solution aqueuse d'acide orthophosphorique dans une première étape de précipitation, précipitation de phosphate de dimagnésium trihydraté comme stabilisant par réaction de solutions aqueuses d'un sel de magnésium et d'acide orthophosphorique en présence d'un composé basique dans une seconde étape de précipitation et séparation subséquente par filtration, séchage et broyage du précipité, procédé caractérisé en ce qu'on ajoute au mélange réactionnel, comme stabilisant supplémentaire, 0,2 à 0,8 % en poids d'acide polyphosphorique, par rapport au phosphate de dicalcium dihydraté précipité dans le mélange réactionnel, l'acide polyphosphorique étant introduit dans la seconde étape de précipitation vers la fin de la précipitation du phosphate de dimagnésium trihydraté.

2. Procédé selon la revendication 1, caractérisé par le fait que l'on utilise de l'acide polyphosphorique ayant une teneur en phosphore correspondant à 78 % en poids de pentoxyde de phosphore.

3. Procédé selon la revendication 1 ou 2, caractérisé par le fait qu'on introduit dans un réacteur des quantités approximativement stoechiométriques de la suspension de carbonate de calcium et de la solution d'acide orthophosphorique et l'on maintient la réaction à des températures n'excédant pas 50°C dans un domaine de pH de 2,2 à 2,6 ; en ce qu'on élève à 5,6 jusqu'à 5,8, à l'aide d'une lessive de soude le domaine de pH du mélange réactionnel et en introduisant simultanément des quantités stoechiométriques de solutions aqueuses de sel de magnésium, d'acide orthophosphorique et de lessive de soude, on précipite du phosphate de dimagnésium trihydraté en une quantité de 2 à 4 % en poids, % calculé sur le phosphate dimagnésium dihydraté précipité dans le mélange réactionnel à un pH de 5,6 à 6,0 ; en ce qu'on ajoute vers la fin de cette précipitation au mélange réactionnel 0,2 à 0,8 % en poids d'acide polyphosphorique ; % calculé sur la base du phosphate dicalcique dihydraté précipité dans le mélange réactionnel, on continue à agiter durant 2 à 3 minutes encore le mélange réactionnel et l'on porte simultanément, à l'aide d'une lessive de soude le pH au point de neutralité, en ce qu'on sépare du mélange réactionnel le produit précipité, on sèche ce produit et on le fragmente à la grosseur des grains usuelle pour l'utilisation dans des pâtes dentifrices.

4. Procédé selon la revendication 3, caractérisé en ce qu'on diminue la quantité stoechiométrique d'acide orthophosphorique à introduire dans la seconde étape de précipitation, selon l'acidité équivalente de la quantité d'acide polyphosphorique à ajouter.

5. Procédé selon l'une au moins des revendications précédentes, caractérisé en ce que, pour le refroidissement, le réacteur est muni d'une double enveloppe.
